(19) Europäisches Patentamt
European Patent Office
Office européen des brevets

(11) Veröffentlichungsnummer : **0 530 639 B1**

(12) # EUROPÄISCHE PATENTSCHRIFT

(45) Veröffentlichungstag der Patentschrift : **17.08.94 Patentblatt 94/33**

(51) Int. Cl.[5] : **C07D 401/12,** A61K 31/47, C07D 417/12

(21) Anmeldenummer : **92114428.3**

(22) Anmeldetag : **25.08.92**

(54) **Heterocyclisch substituierte Chinolylmethoxy-Phenylacetamide als Lipoxygenasehemmer.**

(30) Priorität : **06.09.91 DE 4129742**

(43) Veröffentlichungstag der Anmeldung : **10.03.93 Patentblatt 93/10**

(45) Bekanntmachung des Hinweises auf die Patenterteilung : **17.08.94 Patentblatt 94/33**

(84) Benannte Vertragsstaaten : **AT BE CH DE DK ES FR GB GR IE IT LI LU MC NL PT SE**

(56) Entgegenhaltungen : **EP-A- 0 344 519** **EP-A- 0 399 291**

(73) Patentinhaber : **BAYER AG**
**D-51368 Leverkusen (DE)**

(72) Erfinder : **Raddatz, Siegfried, Dr.**
**Jakob-Böhme-Strasse 21**
**W-5000 Köln 80 (DE)**
Erfinder : **Mohrs, Klaus-Helmut**
**Wildsteig 24**
**W-5600 Wuppertal (DE)**
Erfinder : **Matzke, Michael, Dr.**
**Am Ringelbusch 15**
**W-5600 Wuppertal (DE)**
Erfinder : **Fruchtmann, Romanis**
**Weidenpescher Strasse 14**
**W-5000 Köln 60 (DE)**
Erfinder : **Hatzelmann, Armin, Dr.**
**Alter Wall 3**
**W-7750 Konstanz (DE)**
Erfinder : **Kohlsdorfer, Christian, Dr.**
**Franz-Stryck-Strasse 15**
**W-5042 Erftstadt (DE)**
Erfinder : **Müller-Peddinghaus, Reiner, Prof. Dr.**
**Klutstein 22a**
**W-5060 Bergisch Gladbach 2 (DE)**
Erfinder : **Theisen-Popp, Dr.**
**Horbacher Strasse 368**
**W-5100 Aachen (DE)**

EP 0 530 639 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft heterocyclisch substituierte Chinolylmethoxy-Phenylacetamide, ein Verfahren zu ihrer Herstellung und ihre Verwendung in Arzneimitteln.

Es ist bereits bekannt, daß Chinolylmethoxyphenylacylsulfonamide und -cyanamide eine lipoxygenaseinhibierende Wirkung besitzen [vgl. EP 399 291].

Außerdem sind aus der EP 344 519 4-(Chinolin-2-yl-methoxy)phenylessigsäure-derivate bekannt.

Die vorliegende Erfindung betrifft jetzt heterocyclisch substituierte Chinolylmethoxy-Phenylacetamide der allgemeinen Formel (I)

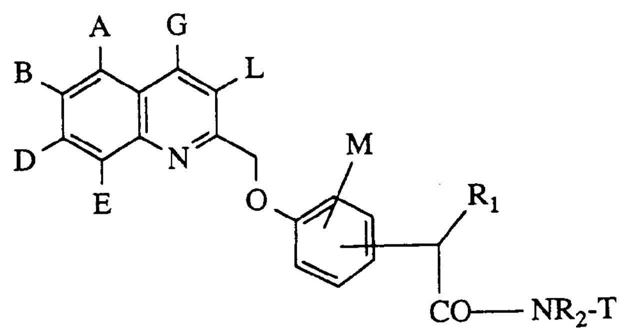

(I),

in welcher

A, B, D, E, G, L und M gleich oder verschieden sind und für Wasserstoff, Hydroxy, Halogen, Cyano, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder für Aryl mit 6 bis 10 Kohlenstoffatomen stehen, das gegebenenfalls durch Halogen, Hydroxy, Nitro oder Cyano substituiert ist,

$R^1$ für geradkettiges oder verzweigtes Alkyl mit bis zu 10 Kohlenstoffatomen steht, das gegebenenfalls durch Aryl mit 6 bis 10 Kohlenstoffatomen oder durch Cycloalkyl mit 3 bis 8 Kohlenstoffatomen substituiert ist, oder für Cycloalkyl oder -alkenyl mit 3 bis 12 Kohlenstoffatomen steht,

$R^2$ für Wasserstoff oder für geradkettiges oder verzweigtes Alkyl mit bis zu 8 Kohlenstoffatomen steht,

T für einen gesättigten oder ungesättigten 5 bis 7-gliedrigen Heterocyclus mit bis zu 4 Heteroatomen aus der Reihe S, O oder N steht, an den gegebenenfalls ein gesättigter oder ungesättigter Heterocyclus oder Carbocyclus ankondensiert ist, und die gegebenenfalls durch geradkettiges oder verzweigtes Alkyl mit bis zu 8 Kohlenstoffatomen, Halogen, Benzyloxy oder durch eine Gruppe der Formel $-NR^3R^4$ oder $-SO_2R^5$ substituiert sind,

worin

$R^3$ und $R^4$ gleich oder verschieden sind und Wasserstoff, Phenyl, Benzyl oder geradkettiges oder verzweigtes Alkyl mit bis zu 8 Kohlenstoffatomen bedeuten

und

$R^5$ Cycloalkyl mit 3 bis 12 Kohlenstoffatomen oder Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert sind, oder geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann,

und deren Salze.

Bevorzugt werden 5- bis 7-gliedrige Heterocyclen, substituiert oder unsubstituiert mit bis zu 4 Heteroatomen aus der Reihe S, N oder O, an denen gegebenenfalls ein weiterer Heterocyclus und/oder ein Cyclohexyl-, Phenyl- oder Naphthylring ankondensiert sind wie beispielsweise Naphthylpyridyl, 1,2,4-Triazinyl, Fluoranthrenyl, Pyrazolopyrimidyl, Imidazolyl, Indolyl, Isochinolyl, Isothiazolyl, Tetrazolyl, Thiazolyl, Thiazolinyl, Isoxazolyl, 1,2,4-Triazolyl, 1,3,4-Thiadiazolyl, Benzimidazolyl, 2,1,3-Benzothiadiazolyl, Benzthiazolyl, Pyridimidyl, Chinolyl, Benzoxazolyl, Pyrazinyl, Pyrryl, Thienyl oder Furyl.

Im Rahmen der vorliegenden Erfindung werden physiologisch unbedenkliche Salze bevorzugt. Physiologisch unbedenkliche Salze der Chinolylmethoxy-Phenylacetamide können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze von Metallen, bevorzugt einwertigen Metallen, und die Ammoniumsalze. Bevorzugt werden Alkalimetalle wie Natrium-, Kaliumsalze und Ammoniumsalze.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

A, B, D, E, G, L und M gleich oder verschieden sind und
für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder
für Phenyl stehen, das gegebenenfalls durch Fluor, Chlor, Brom, Hydroxy, Nitro oder Cyano substituiert ist,

$R^1$ für geradkettiges oder verzweigtes Alkyl mit bis zu 8 Kohlenstoffatomen steht, das gegebenenfalls durch Phenyl, Cyclopentyl, Cyclohexyl oder Cycloheptyl substituiert ist,
für Cyclobutyl, Cyclopentyl, Cyclohexyl, Cycloheptyl oder Cyclooctyl steht,

$R^2$ für Wasserstoff oder für geradkettiges oder verzweigtes Alkyl mit bis zu 6 Kohlenstoffatomen steht,

T für Pyridyl, Triazolyl, Tetrazolyl, Pyrryl, Furyl, Thienyl, Thiazolyl, Thiadiazolyl, Thiazolinyl oder Imidazolyl steht, die gegebenenfalls durch geradkettiges oder verzweigtes Alkyl mit bis zu 6 Kohlenstoffatomen, Fluor, Chlor, Brom oder durch eine Gruppe der Formel $-NR^3R^4$ oder $-SO_2R^5$ substituiert sind,
worin

$R^3$ und $R^4$ gleich oder verschieden sind und Wasserstoff oder geradkettiges oder verzweigtes Alkyl mit bis zu 6 Kohlenstoffatomen bedeuten

und

$R^5$ Cyclobutyl, Cyclopentyl, Cyclohexyl, Cycloheptyl, Cyclooctyl oder Phenyl bedeutet, die gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann,

und deren Salze.

Besonders bevorzugt sind Verbindungen der allgemeinen Formel (I),
in welcher

A, B, D, E, G, L und M gleich oder verschieden sind und
für Wasserstoff, Hydroxy, Fluor, Chlor, Brom oder geradkettiges oder verzweigtes Alkyl mit bis zu 4 Kohlenstoffatomen stehen,

$R^1$ für geradkettiges oder verzweigtes Alkyl mit bis zu 6 Kohlenstoffatomen steht, das gegebenenfalls durch Phenyl oder Cyclohexyl substituiert ist,
für Cyclobutyl, Cyclopentyl, Cyclohexyl, Cycloheptyl oder Cyclooctyl steht,

$R^2$ für Wasserstoff oder für geradkettiges oder verzweigtes Alkyl mit bis zu 4 Kohlenstoffatomen steht,

T für Pyridyl, Triazolyl, Tetrazolyl, Thiazolyl, Thiadiazolyl oder Thiazolinyl steht, die gegebenenfalls durch geradkettiges oder verzweigtes Alkyl mit bis zu 4 Kohlenstoffatomen, Fluor, Chlor, Amino oder durch eine Gruppe der Formel $-SO_2R^5$ substituiert sind,

worin

$R^5$ Cyclopentyl, Cyclohexyl, Cycloheptyl, Cyclooctyl oder Phenyl bedeutet, die gegebenenfalls durch Fluor, Chlor, Brom, Jod, Methoxy oder Trifluormethyl substituiert ist, oder geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann,

und deren Salze.

Ganz besonders bevorzugt sind Verbindungen der Formel (I), in welcher A, B, D, E, G, L und M für Wasserstoff stehen. Darüber hinaus sind solche Verbindungen ganz besonders bevorzugt, in denen die Gruppe $-CHR^1-CO-NR^2-T$ in 4-Stellung zum Chinolylmethoxyrest steht.

Außerdem wurde ein Verfahren zur Herstellung der erfindungsgemäßen Verbindungen der allgemeinen Formel (I) gefunden, dadurch gekennzeichnet, daß man

Verbindungen der allgemeinen Formel (II)

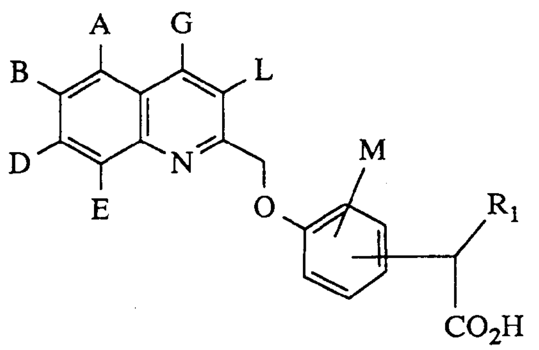

(II)

in welcher

A, B, D, E, G, L, M und $R^1$ die oben angegebene Bedeutung haben,

mit Verbindungen der allgemeinen Formel (III)

$H_2N-T$ (III)

in welcher

T die oben angegebene Bedeutung hat,

in inerten Lösemitteln in Anwesenheit einer Base und/oder eines Hilfsstoffes und gegebenenfalls unter Aktivierung der Carbonsäurefunktion amidiert,

und im Fall der Enantiomeren die entsprechenden enantiomerenreinen Säuren (II) zuvor trennt,

wobei die Substituenten A, B, D, E, G, L, M und $R^1$ gegebenenfalls nach üblichen Methoden variiert werden können.

Das erfindungsgemäße Verfahren kann durch folgendes Formelschema beispielhaft erläutert werden:

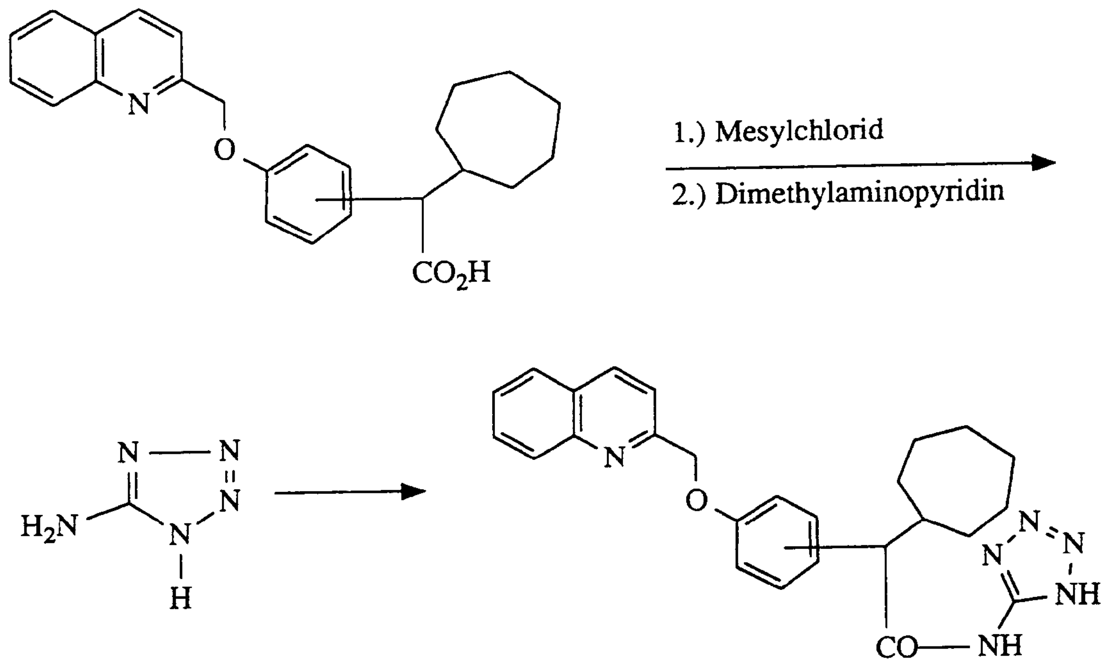


Die Amidierung erfolgt im allgemeinen in inerten Lösemitteln in Anwesenheit einer Base und eines Dehydratisierungsmittels.

Als Lösemittel für das erfindungsgemäße Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimehylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Tetrahydrofuran.

Als Basen für das erfindungsgemäße Verfahren eignen sich die üblichen basischen Verbindungen. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, 2-Dimethylaminopyridin, Triethylamin oder N-Methylpiperidin. Bevorzugt sind Triethylamin und Dimethylaminopyridin.

Bei der Durchführung der Amidierung wird die Base im allgemeinen in einer Menge von 1 bis 3 Mol, bevorzugt von 1 bis 1,5 Mol, bezogen auf 1 Mol der Carbonsäure (II), eingesetzt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Amidierung wird im allgemeinen in einem Temperaturbereich von 0°C bis 150°C, bevorzugt bei 25°C bis 40°C, durchgeführt.

Als Hilfsstoffe werden im allgemeinen Dehydratisierungsmittel, wie sie aus der Peptidchemie literaturbekannt sind, eingesetzt.

Als Dehydratisierungsreagenzien eignen sich Carbodiimide wie beispielsweise Di-isopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexylfluorophosphat oder Phosphonsäurediphenylesteramid oder Methansulfonsäurechlorid, gegebenenfalls in Anwesenheit von Basen wie Triethylamin, Dimethylaminopyridin oder N-Ethylmorpholin oder N-Methylpiperidin oder Dicyclohexylcarbodiimid und N-Hydroxysuccinimid [vgl. J.C. Sheehan, S.L. LEdis, J. Am. Chem. Soc. 95, 875 (1973); F.E. Freeman et al., J. Biol. Chem. 225, 507 (1982) und N.B. Benoiton, K. Kluroda, Int. Pept. Prot. Res. 13, 403 (1979), 17, 187 (1981)].

Die Verbindungen der allgemeinen Formel (II) sind größtenteils bekannt [vgl. z.B. EP 399 291; EP 181 568] oder können nach üblicher Methode hergestellt werden, indem man Verbindungen der allgemeinen Formel (IV)

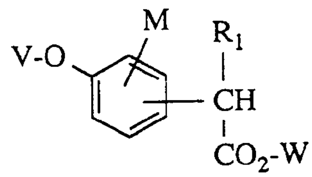

(IV)

in welcher

$R^1$ die oben angegebene Bedeutung hat,

V für eine übliche Hydroxyschutzgruppe, vorzugsweise für Benzyl oder tert.Butyl steht und

W für einen $C_1$-$C_6$-Alkylrest steht,

nach Abspaltung der Schutzgruppe V mit 2-Halogenmethylchinolinen der allgemeinen Formel (V)

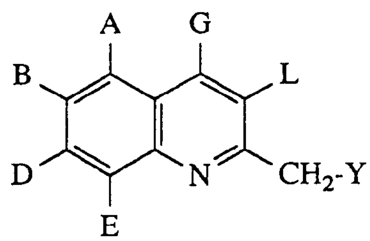

(V),

in welcher

A, B, D, E, G und L die oben angegebene Bedeutung haben, und

Y für Halogen, insbesondere für Chlor oder Brom, steht,

in inerten Lösemitteln zunächst verethert und anschließend verseift, wobei auch auf den Stufen der allgemeinen Formel (IV) und (V) jederzeit die Substituenten A, B, D, E, G, L und M nach üblicher Methode variiert werden können.

Die Abspaltung der Schutzgruppen aus den entsprechenden Ethern (IV) erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley & Sons, 1981, New York].

Die Veretherung kann in inerten organischen Lösemitteln gegebenenfalls in Anwesenheit einer Base durchgeführt werden. Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie z.B. Natriumhydroxid oder Kaliumhydroxid, Erdalkalihydroxide wie z.B. Bariumhydroxid, Alkalicarbonate wie Natriumcarbonat oder Kaliumcarbonat, Erdalkalicarbonate wie Calciumcarbonat, oder organische Amine (Trialkyl($C_1$-$C_6$)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5 Mol, bevorzugt 1 bis 2 Mol Halogenid (V), bezogen auf 1 Mol des Reaktionspartners, ein. Die Base wird im allgemeinen in einer Menge von 0,5 bis 5 Mol, bevorzugt von 1 bis 3 Mol, bezogen auf das Halogenid, eingesetzt.

Die Verbindungen der allgemeinen Formel (IV) sind an sich bekannt oder können nach üblicher Methode hergestellt werden [vgl. J. Org. Chem. 31, 2658 (1966)].

Ebenfalls sind die Verbindungen der allgemeinen Formel (V) und ihre Darstellung bekannt [vgl. Chem. Ber. 120, 649 (1987)].

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C, durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 Mol, bevorzugt von 1 bis 1,5 Mol, bezogen auf 1 Mol des Esters eingesetzt. Besonders bevorzugt verwendet man molare Mengen der Reaktanden.

Die heterocyclischen Amine der allgemeinen Formel (III) sind bekannt oder können nach üblicher Methode hergestellt werden [vgl. z.B. Beilstein 22, 428; Beilstein 27, 155].

Die erfindungsgemäßen heterocyclischen Chinolylmethoxy-Phenylacetamide können als Wirkstoffe in Arzneimitteln eingesetzt werden. Die Stoffe können als Hemmer von enzymatischen Reaktionen im Rahmen des Arachidonsäurestoffwechsels, insbesondere der Lipoxygenase, wirken.

Sie sind somit bevorzugt zur Behandlung und Verhütung von Erkrankungen der Atemwege wie Allergien/Asthma, Bronchitis, Emphysem, Schocklunge, pulmonaler Hypertonie, Entzündungen/Rheuma und Ödemen, Thrombosen und Tromboembolien, Ischämien (periphere, cardiale, cerebrale Durchblutungsstörungen), Herz- und Hirninfarkten, Angina pectoris, Arteriosklerose, bei Gewebstransplatationen, Dermatosen wie Psoriasis, entzündliche Dermatosen und zur Cytoprotection im Gastrointestinaltrakt geeignet.

Die erfindungsgemäßen heterocyclischen Chinolylmethoxy-Phenylacetamide können sowohl in der Humanmedizin als auch in der Veterinärmedizin angewendet werden.

Die pharmakologischen Wirkungen der erfindungsgemäßen Substanzen werden durch folgende Methode bestimmt:

Als Maß für die Lipoxyenase-Hemmung wurde die Freisetzung von Leukotrien $B_4$ ($LTB_4$) an polymorphkernigen Humanleukozyten (PMN) nach Zugabe von Substanzen und Ca-Ionophor mittels reverse phase HPLC nach Borgeat, P. et al., Proc. Nat. Acad Sci, 76, 2148 - 2152 (1979), bestimmt.

In der Tabelle 1 sind beispielhaft die nach diesem Test erzielten Werte einiger erfindungsgemäßer Verbindungen aufgeführt:

Tabelle 1:

| Beispiel-Nr. | 5-LO $IC_{50}$ (µmol/l) |
|---|---|
| 1 | $5 \times 10^{-7}$ |
| 2 | $2,6 \times 10^{-7}$ |
| 3 | $2,9 \times 10^{-7}$ |
| 4 | $4,3 \times 10^{-7}$ |

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung, vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

2-[4-(Chinolin-2-ylmethoxy)phenyl]-2-cycloheptylessigsäure

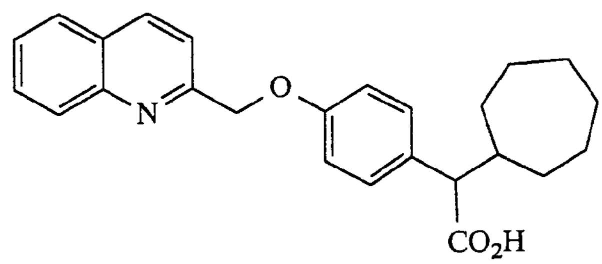

11 g (27 mmol) 2-[4-(Chinolin-2-yl-methoxy)phenyl]-2-cycloheptyl-essigsäuremethylester in 200 ml Methanol und 55,4 ml 1 molarer Natriumhydroxydlösung werden 10 h auf Rückfluß erwärmt. Nach Abkühlung wird mit konzentrierter Salzsäure angesäuert, das ausgefallene Produkt abgesaugt und getrocknet.
Ausbeute: 9,3 g (87% der Theorie)
Festpunkt: 176°C

Herstellungsbeispiele

Beispiel 1

N-(2-Pyridyl)-2-[4-(chinolin-2-yl-methoxy)phenyl]-2-cycloheptyl-acetamid

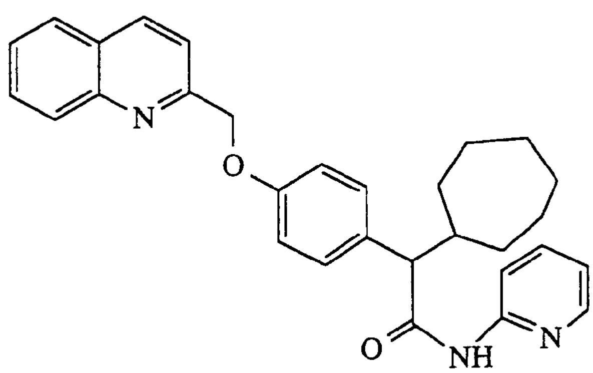

Bei Raumtemperatur werden 2 g (0,005 mol) der Verbindung aus Beispiel I in 20 ml getrocknetem THF

suspendiert, unter Argon auf 0°C im Eisbad abgekühlt und unter Rühren mit 1,4 ml = 1,0 g (0,01 mol) Triethylamin versetzt. Dabei entsteht eine klare Lösung. Nach Zugabe von 0,39 ml = 0,58 g (0,005 mol) Methansulfonsäurechlorid bei 0°C läßt man 2 h im Eisbad rühren. Dabei fallen farblose Kristalle an. Hierzu gibt man unter Rühren 0,56 g (0,006mol) 2-Aminopyridin und 1,2 g (0,01 mol) Dimethylaminopyridin, gelöst in 10 ml trockenem THF. Die Suspension färbt sich anfangs gelb, wird aber später wieder farblos. Man läßt den Ansatz über Nacht bei Raumtemperatur rühren. Nach Zusatz von 2 ml 2 N Essigsäure dampft man alles im Vakuum zur Trockene ein. Den Rückstand nimmt man in 70 ml Toluol auf, schüttelt mit ges. $NaHOC_3$-Lösung und Wasser aus, trocknet die organische Phase mit Natriumsulfat, engt auf ein kleines Volumen ein und trennt den Rückstand säulenchromatographisch (Kieselgel 60, Laufmittel: Dichlormethan: Essigester: Eisessig = 80:10:10).
Ausbeute: 2,7 g (75,3% der Theorie) farblose Kristalle
Fp.: 137°C

Beispiel 2

N-(5-Tetrazolyl)-2-[4-(chinolin-2-yl-methoxy)phenyl]-2-cycloheptyl-acetamid

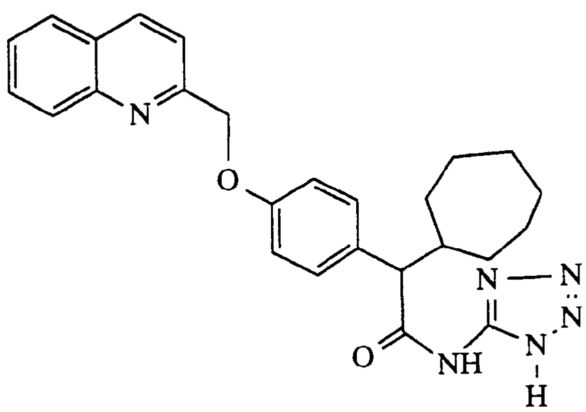

2 g (0,005 mol) der Verbindung aus Beispiel I und 2 ml = 1,5 g (0,015 mol) Triethylamin werden in 30 ml THF gelöst, auf 0°C abgekühlt, tropfenweise mit 0,8 ml = 1,1 g (0,01 mol) Mesylchlorid versetzt, und anschließend werden 1,2 g (0,01 mol) Dimethylaminopyridin und 0,6 g (0,006 mol) 5-Aminotetrazol zugegeben. Man läßt alles über Nacht bei Raumtemperatur rühren, engt im Vakuum zur Trockene ein und rührt den Rückstand mit 10 ml Wasser aus. Den abfiltrierten und getrockneten Rückstand löst mit in wenig Essigester und trennt über Säulenchromatographie (Kieselgel 60, Laufmittel: Toluol, Essigester , Eisessig = 8,1,1).
Ausbeute: 1,8 g (79% der Theorie) farblose Kristalle
Fp.: ca. 255°C (Zers.)

Beispiel 3

N-(2-Thiazolinyl)-2-[4-(chinolin-2-yl-methoxy)phenyl]-2-cycloheptyl-acetamid

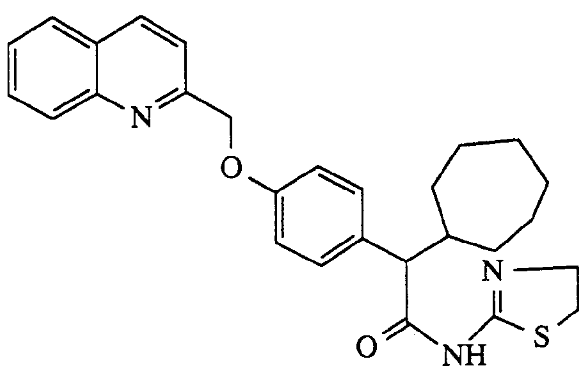

2,0 g (0,005 mol) der Verbindung aus Beispiel I und 1,4 ml = 1,0 g (0,01 mol) Triethylamin werden in 30 ml THF gelöst, auf 0°C abgekühlt, tropfenweise mit 0,4 ml = 0,6 g (0,0052 mol) Mesylchlorid und anschließend mit 0,6 g (0,006 mol) 2-Aminothiazolin und 1,2 g (0,01 mol) Dimethylaminopyridin in 15 ml THF versetzt. Man läßt über Nacht bei Raumtemperatur rühren. Anschließend dampft man das Lösemittel im Vakuum zur Trockene ein, nimmt in 50 ml Dichlormethan auf und schüttelt zweimal mit 15 ml Wasser aus. Nach Trocknen mit Natriumsulfat und Einengen auf ein kleines Volumen trennt man das Gemisch säulenchromatographisch (Kieselgel 60, Laufmittel: Toluol, Essigester, Eisessig = 8/1/1).
Ausbeute: 1,0 g (42% der Theorie) farbloser Schaum
Fp.: ca. 80°C

Beispiel 4

N-(2-Thiazolyl)-2-[4-(chinolin-2-yl-methoxy)phenyl]-2-cycloheptyl-acetamid

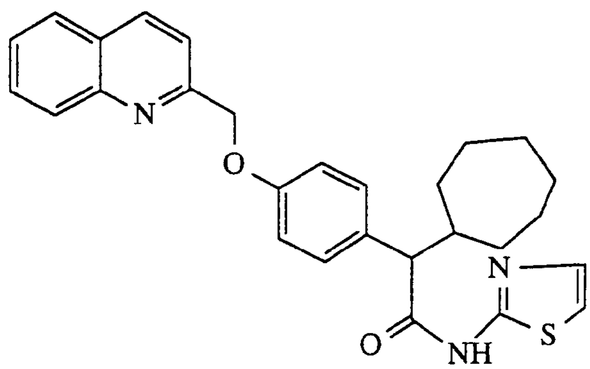

In Analogie zur Vorschrift des Beispiels 3 werden 2,0 g (0,005 mol) der Verbindung aus Beispiel I, 1,4 ml = 1,0 g (0,01 mol) Triethylamin, 0,4 ml = 0,6 g (0,0052 mol) Mesylchlorid, 0,6 g (0,006 mol) 2-Aminothiazol und 1,2 g (0,01 mol) Dimethylamiopyridin zur Herstellung der Titelverbindung umgesetzt.
Ausbeute: 1,8 g (77% der Theorie) farblose Kristalle
Fp.: 166°C

Beispiel 5

N-(5-Cyclohexylsulfonyl-1,3,4-thiadiazolyl-2-yl)-2-[4-(chinolin-2-yl-methoxy)phenyl]-2-cycloheptyl-acetamid

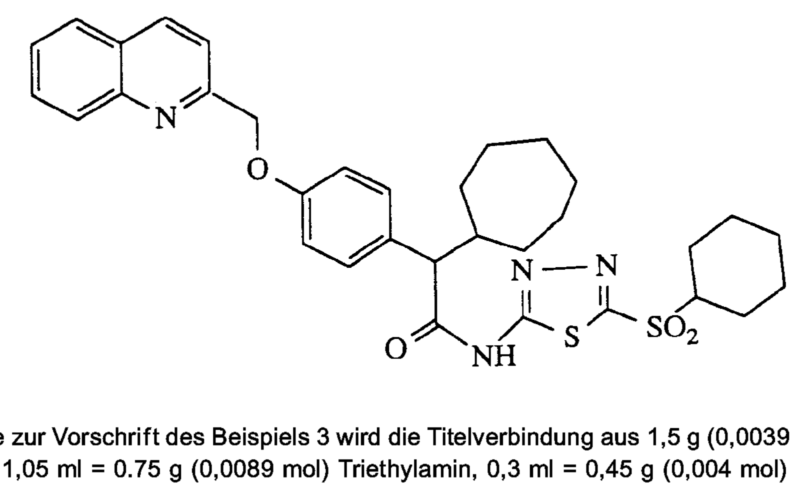

In Analogie zur Vorschrift des Beispiels 3 wird die Titelverbindung aus 1,5 g (0,0039 mol) der Verbindung aus Beispiel I, 1,05 ml = 0.75 g (0,0089 mol) Triethylamin, 0,3 ml = 0,45 g (0,004 mol) Mesylchlorid, 0,96 g (0,004 mol) 2-Amino-5-cyclohexylsulfonyl-1,3,4-thiadiazol und 0,9 g (0,0075 mol) Dimethylaminopyridin hergestellt.
Ausbeute: 0,9 g (37,8% der Theorie) farblose Kristalle
Fp.: 183°C

**Patentansprüche**

**Patentansprüche für folgende Vertragsstaaten : AT, BE, CH, DE, DK, FR, GB, GR, IE, IT, LU, MC, NL, LI, PT, SE**

1. Heterocyclisch substituierte Chinolylmethoxy-Phenylacetamide der allge meinen Formel

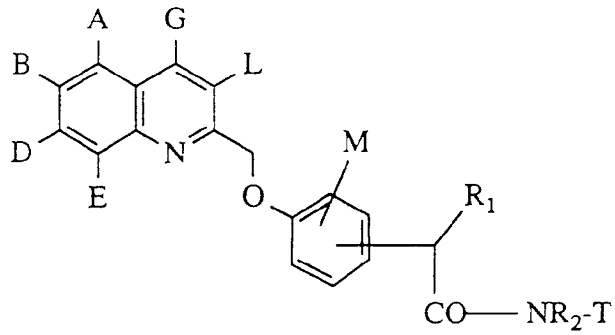

(I),

in welcher

| | |
|---|---|
| A, B, D, E, G, L und M | gleich oder verschieden sind und für Wasserstoff, Hydroxy, Halogen, Cyano, Carboxy, Nitro, Trifluormethyl, Trifluormethoxy oder für geradkettiges oder verzweigtes Alkyl oder Alkoxy mit jeweils bis zu 8 Kohlenstoffatomen stehen, oder für Aryl mit 6 bis 10 Kohlenstoffatomen stehen, das gegebenenfalls durch Halogen, Hydroxy, Nitro oder Cyano substituiert ist, |
| $R^1$ | für geradkettiges oder verzweigtes Alkyl mit bis zu 10 Kohlenstoffatomen steht, das gegebenenfalls durch Aryl mit 6 bis 10 Kohlenstoffatomen oder durch Cycloalkyl mit 3 bis 8 Kohlenstoffatomen substituiert ist, oder für Cycloalkyl oder -alkenyl mit 3 bis 12 Kohlenstoffatomen steht, |
| $R^2$ | für Wasserstoff oder für geradkettiges oder verzweigtes Alkyl mit bis zu 8 Kohlenstoffatomen steht, |
| T | für einen gesättigten oder ungesättigten 5- bis 7-gliedrigen Heterocyclus mit bis zu 4 Heteroatomen aus der Reihe S, O oder N steht, an den gegebenenfalls ein gesättigter oder ungesättigter Heterocyclus oder Carbocyclus ankondensiert ist, und die gegebenenfalls durch geradkettiges oder verzweigtes Alkyl mit bis zu 8 Kohlenstoffatomen, Halogen, Benzyloxy oder durch eine Gruppe der Formel $-NR^3R^4$ oder $-SO_2R^5$ substituiert sind, |

worin

| | |
|---|---|
| $R^3$ und $R^4$ | gleich oder verschieden sind und Wasserstoff, Phenyl, Benzyl oder geradkettiges oder verzweigtes Alkyl mit bis zu 8 Kohlenstoffatomen bedeuten, und |
| $R^5$ | Cycloalkyl mit 3 bis 12 Kohlenstoffatomen oder Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert sind, oder geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann, |

und deren Salze.

2. Heterocyclisch substituierte Chinolylmethoxy-Phenylacetamide nach Anspruch 1, worin

A, B, D, E, G, L und M gleich oder verschieden sind und

für Wasserstoff, Hydroxy, Fluor, Chlor, Brom, Carboxy, Nitro, Trifluormethyl, Trifluormethoxy oder
für geradkettiges oder verzweigtes Alkyl oder Alkoxy mit jeweils bis zu 6 Kohlenstoffatomen stehen, oder
für Phenyl stehen, das gegebenenfalls durch Fluor, Chlor, Brom, Hydroxy, Nitro oder Cyano substituiert ist,

$R^1$ für geradkettiges oder verzweigtes Alkyl mit bis zu 8 Kohlenstoffatomen steht, das gegebenenfalls durch Phenyl, Cyclopentyl, Cyclohexyl oder Cycloheptyl substituiert ist,
für Cyclobutyl, Cyclopentyl, Cyclohexyl, Cycloheptyl oder Cyclooctyl steht,

$R^2$ für Wasserstoff oder für geradkettiges oder verzweigtes Alkyl mit bis zu 6 Kohlenstoffatomen steht,

T für Pyridyl, Triazolyl, Tetrazolyl, Pyrryl, Furyl, Thienyl, Thiazolyl, Thiadiazolyl, Thiazolinyl oder Imidazolyl steht, die gegebenenfalls durch geradkettiges oder verzweigtes Alkyl mit bis zu 6 Kohlenstoffatomen, Fluor, Chlor, Brom oder durch eine Gruppe der Formel $-NR^3R^4$ oder $-SO_2R^5$ substituiert sind,

worin

$R^3$ und $R^4$ gleich oder verschieden sind und Wasserstoff oder geradkettiges oder verzweigtes Alkyl mit bis zu 6 Kohlenstoffatomen bedeuten,
und

$R^5$ Cyclobutyl, Cyclopentyl, Cyclohexyl, Cycloheptyl, Cyclooctyl oder Phenyl bedeutet, die gegebenenfalls durch Fluor, Chlor, Brom, Iod, Cyano oder durch geradkettiges oder verzweigtes Alkyl oder Alkoxy mit jeweils bis zu 6 Kohlenstoffatomen substituiert ist, oder
geradkettiges oder verzweigtes Alkyl mit bis zu 6 Kohlenstoffatomen bedeutet, das gegebenenfalls durch Phenyl substituiert ist, das seinerseits durch Fluor, Chlor, Brom, Trifluormethyl oder durch geradkettiges oder verzweigtes Alkyl oder Alkoxy mit jeweils bis zu 4 Kohlenstoffatomen substituiert sein kann,

und deren Salze.

**3.** Heterocyclisch substituierte Chinolylmethoxy-Phenylacetamide nach Anspruch 1, worin

A, B, D, E, G, L und M gleich oder verschieden sind und
für Wasserstoff, Hydroxy, Fluor, Chlor, Brom oder geradkettiges oder verzweigtes Alkyl mit bis zu 4 Kohlenstoffatomen stehen,

$R^1$ für geradkettiges oder verzweigtes Alkyl mit bis zu 6 Kohlenstoffatomen steht, das gegebenenfalls durch Phenyl oder Cyclohexyl substituiert ist,
für Cyclobutyl, Cyclopentyl, Cyclohexyl, Cycloheptyl oder Cyclooctyl steht,

$R^2$ für Wasserstoff oder für geradkettiges oder verzweigtes Alkyl mit bis zu 4 Kohlenstoffatomen steht,

T für Pyridyl, Triazolyl, Tetrazolyl, Thiazolyl, Thiadiazolyl oder Thiazolinyl steht, die gegebenenfalls durch geradkettiges oder verzweigtes Alkyl mit bis zu 4 Kohlenstoffatomen, Fluor, Chlor, Amino oder durch eine Gruppe der Formel $-SO_2R^5$ substituiert sind,

worin

$R^5$ Cyclopentyl, Cyclohexyl, Cycloheptyl, Cyclooctyl oder Phenyl bedeutet, die gegebenenfalls durch Fluor, Chlor, Brom, Jod, Methoxy oder Trifluormethyl substituiert ist, oder
geradkettiges oder verzweigtes Alkyl mit bis zu 4 Kohlenstoffatomen bedeutet, das gegebenenfalls durch Phenyl substituiert ist, das seinerseits durch Fluor, Chlor, Brom, Methyl oder Methoxy substituiert sein kann,

und deren Salze.

**4.** Heterocyclisch substituierte Chinolylmethoxy-Phenylacetamide nach Anspruch 1, worin die Gruppe $-CHR^1-CO-NR^2-T$ in 4-Stellung zum Chinolylmethoxyrest steht.

5. Heterocyclisch substituierte Chinolylmethoxy-Phenylacetamide nach Anspruch 1 zur Bekämpfung von Krankheiten.

6. Verfahren zur Herstellung von heterocyclisch substituierten Chinolylmethoxy-Phenylacetamiden nach Anspruch 1 - 4, dadurch gekennzeichnet, daß man Verbindungen der allgemeinen Formel

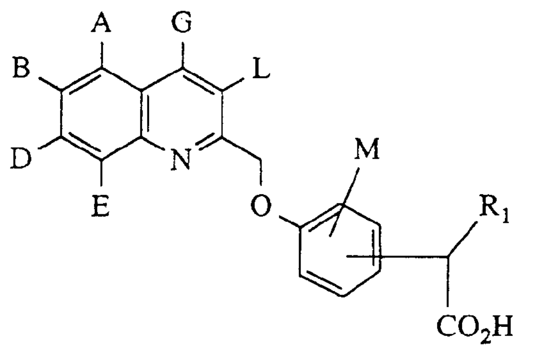

(II)

in welcher
A, B, D, E, G, L, M und $R^1$ die oben angegebene Bedeutung haben,
mit Verbindungen der allgemeinen Formel (III)

$H_2N$-T (III)

in welcher
T die oben angegebene Bedeutung hat,
in inerten Lösemitteln in Anwesenheit einer Base und/oder eines Hilfsstoffes und gegebenenfalls unter Aktivierung der Carbonsäurefunktion amidiert,
und im Fall der Enantiomeren die entsprechenden enantiomerenreinen Säuren (II) zuvor trennt,
wobei die Substituenten A, B, D, E, G, L, M und $R^1$ gegebenenfalls nach üblichen Methoden variiert werden können.

7. Arzneimittel enthaltend heterocyclisch substituierte ChinolylmethoxyPhenylacetamide nach Anspruch 1.

8. Verwendung von heterocyclisch substituierten Chinolylmethoxy-Phenylacetamiden nach Anspruch 1 zur Herstellung von Arzneimitteln.

9. Verwendung von heterocyclisch substituierten Chinolylmethoxy-Phenylacetamiden nach Anspruch 1 zur Herstellung von lipoxygenasehemmenden Arzneimitteln.

**Patentansprüche für folgenden Vertragsstaat : ES**

1. Verfahren zur Herstellung von heterocyclisch substituierten Chinolylmethoxy-Phenylacetamiden der allgemeinen Formel

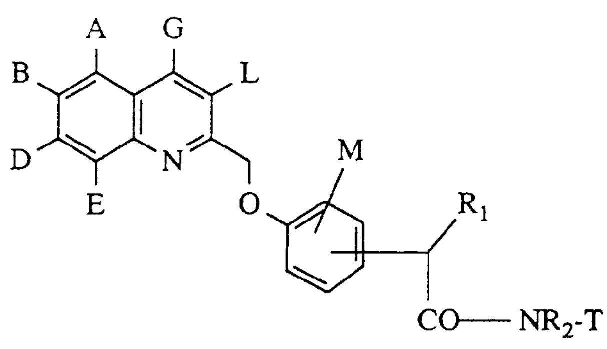

(I),

in welcher

A, B, D, E, G, L und M gleich oder verschieden sind und
für Wasserstoff, Hydroxy, Halogen, Cyano, Carboxy, Nitro, Trifluormethyl, Trifluormethoxy oder
für geradkettiges oder verzweigtes Alkyl oder Alkoxy mit jeweils bis zu 8 Kohlenstoffatomen stehen, oder
für Aryl mit 6 bis 10 Kohlenstoffatomen stehen, das gegebenenfalls durch Halogen, Hydroxy, Nitro oder Cyano substituiert ist,

$R^1$ für geradkettiges oder verzweigtes Alkyl mit bis zu 10 Kohlenstoffatomen steht, das gegebenenfalls durch Aryl mit 6 bis 10 Kohlenstoffatomen oder durch Cycloalkyl mit 3 bis 8 Kohlenstoffatomen substituiert ist, oder
für Cycloalkyl oder -alkenyl mit 3 bis 12 Kohlenstoffatomen steht,

$R^2$ für Wasserstoff oder für geradkettiges oder verzweigtes Alkyl mit bis zu 8 Kohlenstoffatomen steht,

T für einen gesättigten oder ungesättigten 5- bis 7-gliedrigen Heterocyclus mit bis zu 4 Heteroatomen aus der Reihe S, O oder N steht, an den gegebenenfalls ein gesättigter oder ungesättigter Heterocyclus oder Carbocyclus ankondensiert ist, und die gegebenenfalls durch geradkettiges oder verzweigtes Alkyl mit bis zu 8 Kohlenstoffatomen, Halogen, Benzyloxy oder durch eine Gruppe der Formel $-NR^3R^4$ oder $-SO_2R^5$ substituiert sind,

worin

$R^3$ und $R^4$ gleich oder verschieden sind und Wasserstoff, Phenyl, Benzyl oder geradkettiges oder verzweigtes Alkyl mit bis zu 8 Kohlenstoffatomen bedeuten,

und

$R^5$ Cycloalkyl mit 3 bis 12 Kohlenstoffatomen oder Aryl mit 6 bis 10 Kohlenstoffatomen bedeutet, das gegebenenfalls bis zu 2-fach gleich oder verschieden durch Halogen, Cyano, Hydroxy, Nitro, Trifluormethyl, Trifluormethoxy, Trifluormethylthio, oder durch geradkettiges oder verzweigtes Alkyl oder Alkoxy mit jeweils bis zu 8 Kohlenstoffatomen substituiert sind, oder
geradkettiges oder verzweigtes Alkyl mit bis zu 8 Kohlenstoffatomen bedeutet, das gegebenenfalls durch Phenyl substituiert ist, das seinerseits durch Halogen, Cyano, Nitro, Trifluormethyl, Trifluormethoxy, Trifluormethylthio, Hydroxy oder durch geradkettiges oder verzweigtes Alkyl oder Alkoxy mit jeweils bis zu 6 Kohlenstoffatomen substituiert sein kann,

und deren Salze,
dadurch gekennzeichnet, daß man Verbindungen der allgemeinen Formel

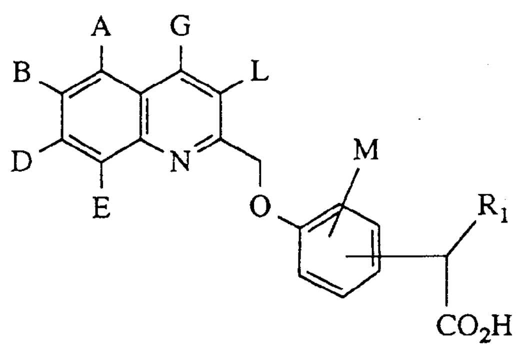

(II)

in welcher
A, B, D, E, G, L, M und $R^1$ die oben angegebene Bedeutung haben,
mit Verbindungen der allgemeinen Formel (III)

$H_2N-T$ (III)

in welcher
T die oben angegebene Bedeutung hat,
in inerten Lösemitteln in Anwesenheit einer Base und/oder eines Hilfsstoffes und gegebenenfalls unter Aktivierung der Carbonsäurefunktion amidiert,
und im Fall der Enantiomeren die entsprechenden enantiomerenreinen Säuren (II) zuvor trennt,

wobei die Substituenten A, B, D, E, G, L, M und $R^1$ gegebenenfalls nach üblichen Methoden variiert werden können.

## Claims

**Claims for the following Contracting States : AT, BE, CH, DE, DK, FR, GB, GR, IE, IT, LU, MC, NL, LI, PT, SE**

1. Heterocyclically substituted quinoiylmethoxy-phenylacetamides of the general formula

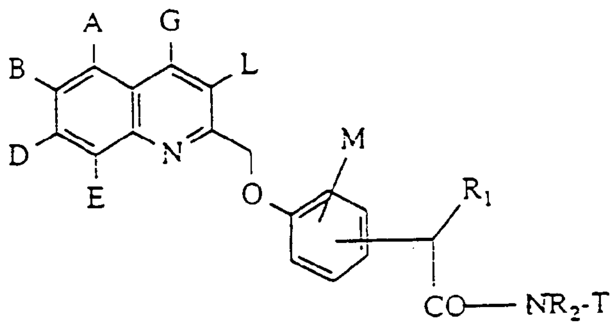

(I),

in which

A, B, D, E, G, L and M are identical or different and represent hydrogen, hydroxyl, halogen, cyano, carboxyl, nitro, trifluoromethyl, trifluoromethoxy or represent straight-chain or branched alkyl or alkoxy each having up to 8 carbon atoms, or represent aryl having 6 to 10 carbon atoms, which is optionally substituted by halogen, hydroxyl, nitro or cyano,

$R^1$ represents straight-chain or branched alkyl having up to 10 carbon atoms, which is optionally substituted by aryl having 6 to 10 carbon atoms or by cycloalkyl having 3 to 8 carbon atoms, or represents cycloalkyl or -alkenyl having 3 to 12 carbon atoms,

$R^2$ represents hydrogen or straight-chain or branched alkyl having up to 8 carbon atoms,

T represents a saturated or unsaturated 5- to 7-membered heterocycle having up to 4 heteroatoms from the series comprising S, O and N, to which a saturated or unsaturated heterocycle or carbocycle is optionally fused, and which are optionally substituted by straight-chain or branched alkyl having up to 8 carbon atoms, halogen, benzyloxy or by a group of the formula $-NR^3R^4$ or $-SO_2R^5$,

in which

$R^3$ and $R^4$ are identical or different and denote hydrogen, phenyl, benzyl or straightchain or branched alkyl having up to 8 carbon atoms

and

$R^5$ denotes cycloalkyl having 3 to 12 carbon atoms or aryl having 6 to 10 carbon atoms, which is optionally monosubstituted or disubstituted by identical or different halogen, cyano, hydroxyl, nitro, trifluoromethyl, trifluoromethoxy or trifluoromethylthio substituents, or by straight-chain or branched alkyl or alkoxy each having up to 8 carbon atoms, or denotes straight-chain or branched alkyl having up to 8 carbon atoms, which is optionally substituted by phenyl which in turn can be substituted by halogen, cyano, nitro, trifluoromethyl, trifluoromethoxy, trifluoromethylthio, hydroxyl or by straight-chain or branched alkyl or alkoxy each having up to 6 carbon atoms,

and their salts.

2. Heterocyclically substituted quinolylmethoxy-phenylacetamides according to Claim 1, in which
A, B, D, E, G, L and M are identical or different and represent hydrogen, hydroxyl, fluorine, chlorine, bromine, carboxyl, nitro, trifluoromethyl, trifluoromethoxy or represent straight-chain or branched alkyl or alkoxy each having up to 6 carbon atoms, or
represent phenyl which is optionally substituted by fluorine, chlorine, bromine, hydroxyl, nitro or cyano,
$R^1$ represents straight-chain or branched alkyl having up to 8 carbon atoms, which is optionally substituted by phenyl, cyclopentyl, cyclohexyl or cycloheptyl,
represents cyclobutyl, cyclopentyl, cyclohexyl, cycloheptyl or cyclooctyl,
$R^2$ represents hydrogen or straight-chain or branched alkyl having up to 6 carbon atoms,
T represents pyridyl, triazolyl, tetrazolyl, pyrryl, furyl, thienyl, thiazolyl, thiadiazolyl, thiazolinyl or imidazolyl, each of which is optionally substituted by straight-chain or branched alkyl having up to 6 carbon atoms, fluorine, chlorine, bromine or by a group of the formula $-NR^3R^4$ or $-SO_2R^5$,
in which
$R^3$ and $R^4$ are identical or different and denote hydrogen or straight-chain or branched alkyl having up to 6 carbon atoms
and
$R^5$ denotes cyclobutyl, cyclopentyl, cyclohexyl, cycloheptyl, cyclooctyl or phenyl, each of which is optionally substituted by fluorine, chlorine, bromine, iodine, cyano or by straight-chain or branched alkyl or alkoxy each having up to 6 carbon atoms, or
denotes straight-chain or branched alkyl having up to 6 carbon atoms, which is optionally substituted by phenyl which in turn can be substituted by fluorine, chlorine, bromine, trifluoromethyl or by straight-chain or branched alkyl or alkoxy each having up to 4 carbon atoms,
and their salts.

3. Heterocyclically substituted quinolylmethoxy-phenylacetamides according to Claim 1, in which
A, B, D, E, G, L and M are identical or different and represent hydrogen, hydroxyl, fluorine, chlorine, bromine or straight-chain or branched alkyl having up to 4 carbon atoms,
$R^1$ represents straight-chain or branched alkyl having up to 6 carbon atoms, which is optionally substituted by phenyl or cyclohexyl,
represents cyclobutyl, cyclopentyl, cyclohexyl, cycloheptyl or cyclooctyl,
$R^2$ represents hydrogen or straight-chain or branched alkyl having up to 4 carbon atoms,
T represents pyridyl, triazolyl, tetrazolyl, thiazolyl, thiadiazolyl or thiazolinyl, each of which is optionally substituted by straight-chain or branched alkyl having up to 4 carbon atoms, fluorine, chlorine, amino or by a group of the formula $-SO_2R^5$,
in which
$R^5$ denotes cyclopentyl, cyclohexyl, cycloheptyl, cyclooctyl or phenyl, each of which is optionally substituted by fluorine, chlorine, bromine, iodine, methoxy or trifluoromethyl, or denotes straight-chain or branched alkyl having to 4 carbon atoms, which is optionally substituted by phenyl which in turn can be substituted by fluorine, chlorine, bromine, methyl or methoxy,
and their salts.

4. Heterocyclically substituted quinolylmethoxy-phenyl acetamides according to Claim 1, in which the group $-CHR^1-CO-NR^2-T$ is in the 4-position to the quinolylmethoxy radical.

5. Heterocyclically substituted quinolylmethoxy-phenyl acetamides according to Claim 1 for controlling diseases.

6. Process for the preparation of heterocyclically substituted quinolylmethoxy-phenylacetamides according to Claims 1-4, characterized in that compounds of the general formula

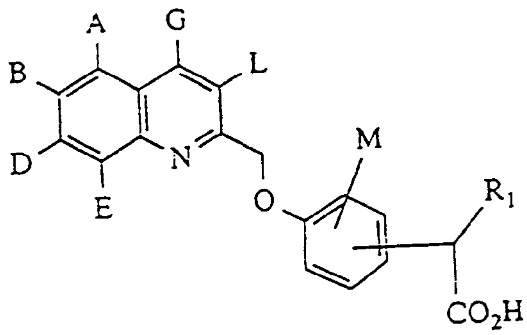

(II)

in which
A, B, D, E, G, L, M and $R^1$ have the abovementioned meaning,
are amidated with compounds of the general formula (III)

$H_2N$-T (III)

in which
T has the abovementioned meaning,
in inert solvents, in the presence of a base and/or of an auxiliary and optionally with activation of the carboxylic acid function,
and in the case of the enantiomers, the corresponding enantiomerically pure acids (II) are previously separated,
where the substituents A, B, D, E, G, L, M and $R^1$ can optionally be varied according to customary methods.

7. Medicament containing heterocyclically substituted quinolylmethoxy-phenylacetamides according to Claim 1.

8. Use of heterocyclically substituted quinolylmethoxyphenylacetamides according to Claim 1 for the production of medicaments.

9. Use of heterocyclically substituted quinolylmethoxyphenylacetamides according to Claim 1 for the production of lipoxygenase-inhibiting medicaments.

**Claims for the following Contracting State : ES**

1. Process for the preparation of heterocyclically substitutedquinolylmethoxy-phenylacetamides of the general formula

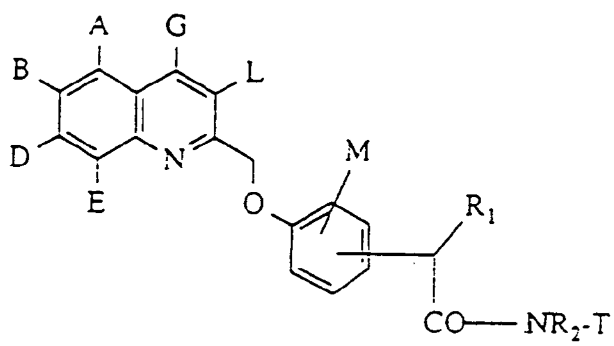

(I)

in which

| | |
|---|---|
| A, B, D, E, G, L and M | are identical or different and represent hydrogen, hydroxyl, halogen, cyano, carboxyl, nitro, trifluoromethyl, trifluoromethoxy or |

represent straight-chain or branched alkyl or alkoxy each having up to 8 carbon atoms, or
represent aryl having 6 to 10 carbon atoms, which is optionally substituted by halogen, hydroxyl, nitro or cyano,

$R^1$ represents straight-chain or branched alkyl having up to 10 carbon atoms, which is optionally substituted by aryl having 6 to 10 carbon atoms or by cycloalkyl having 3 to 8 carbon atoms, or represents cycloalkyl or -alkenyl having 3 to 12 carbon atoms,

$R^2$ represents hydrogen or straight-chain or branched alkyl having up to 8 carbon atoms,

T represents a saturated or unsaturated 5- to 7-membered heterocycle having up to 4 heteroatoms from the series comprising S, O and N, to which a saturated or unsaturated heterocycle or carbocycle is optionally fused and which are optionally substituted by straight-chain or branched alkyl having up to 8 carbon atoms, halogen, benzyloxy or by a group of the formula $-NR^3R^4$ or $-SO_2R^5$,

in which

$R^3$ and $R^4$ are identical or different and denote hydrogen, phenyl, benzyl or straightchain or branched alkyl having up to 8 carbon atoms

and

$R^5$ denotes cycloalkyl having 3 to 12 carbon atoms or aryl having 6 to 10 carbon atoms, which is optionally monosubstituted or disubstituted by identical or different halogen, cyano, hydroxyl, nitro, trifluoromethyl, trifluoromethoxy or trifluoromethylthio substituents, or by straight-chain or branched alkyl or alkoxy each having up to 8 carbon atoms, or
denotes straight-chain or branched alkyl having up to 8 carbon atoms, which is optionally substituted by phenyl which in turn can be substituted by halogen, cyano, nitro, trifluoromethyl, trifluoromethoxy, trifluoromethylthio, hydroxyl or by straight-chain or branched alkyl or alkoxy each having up to 6 carbon atoms,

and their salts,
characterized in that compounds of the general formula

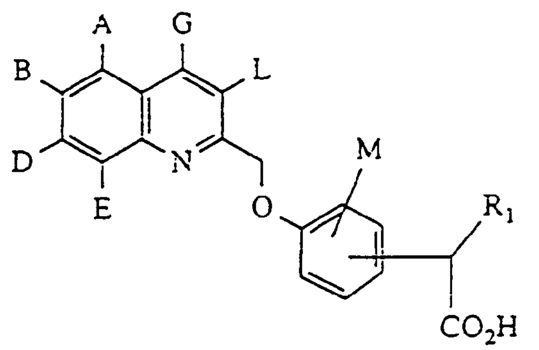

(II)

in which
A, B, D, E, G, L, M and $R^1$ have the abovementioned meaning,
are amidated with compounds of the general formula (III)

$H_2N$-T (III)

in which
T has the abovementioned meaning,
in inert solvents, in the presence of a base and/or of an auxiliary and optionally with activation of the carboxylic acid function,
and in the case of the enantiomers, the corresponding enantiomerically pure acids (II) are previously separated,
where the substituents A, B, D, E, G, L, M and $R^1$ can optionally be varied according to customary methods.

## Revendications

**Revendications pour les Etats contractants suivants : AT, BE, CH, DE, DK, FR, GB, GR, IE, IT, LU, MC, NL, LI, PT, SE**

1. Quinoléylméthoxyphénylacétamides à substituants hétérocycliques de formule générale:

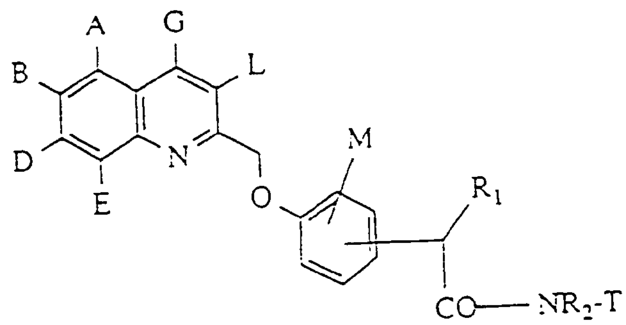

(I),

dans laquelle:
A, B, D, E, G, L et M, ayant des significations identiques ou différentes, représentent chacun:
l'hydrogène, un groupe hydroxy, un halogène, un groupe cyano, carboxy, nitro, trifluorométhyle,trifluorométhoxy, ou bien
un groupe alkyle ou alcoxy à chaîne droite ou ramifiée contenant chacun jusqu'à 8 atomes de carbone, ou bien
un groupe aryle en $C_6$-$C_{10}$ éventuellement substitué par des halogènes, des groupes hydroxy, nitro ou cyano,
$R^1$ représente un groupe alkyle à chaîne droite ou ramifiée contenant jusqu'à 10 atomes de carbone et éventuellement substitué par un groupe aryle en $C_6$-$C_{10}$ ou par un groupe cycloalkyle en $C_3$-$C_8$, ou bien un groupe cycloalkyle ou -alcényle contenant 3 à 12 atomes de carbone,
$R^2$ représente l'hydrogène ou un groupe alkyle à chaîne droite ou ramifiée contenant jusqu'à 8 atomes de carbone,
T représente un hétérocycle saturé ou insaturé de 5 à 7 chaînons contenant jusqu'à 4 hétéroatomes choisis parmi S, O ou N, et condensé le cas échéant avec un hétérocycle ou un carbocycle saturé ou insaturé, ces cycles étant éventuellement substitués par des groupes alkyle à chaîne droite ou ramifiée contenant jusqu'à 8 atomes de carbone, des halogènes, des groupes benzyloxy ou un groupe de formules $-NR^3R^4$ ou $-SO_2R^5$, dans lesquelles :
$R^3$ et $R^4$, ayant des significations identiques ou différentes, représentent chacun l'hydrogène, un groupe phényle, benzyle ou un groupe alkyle à chaîne droite ou ramifiée contenant jusqu'à 8 atomes de carbone, et
$R^5$ représente un groupe cycloalkyle en $C_3$-$C_{12}$ ou aryle en $C_6$-$C_{10}$, portant éventuellement jusqu'à deux substituants identiques ou différents choisis parmi les halogènes, les groupes cyano, hydroxy, nitro, trifluorométhyle, trifluorométhoxy, trifluorométhylthio ou un substituant alkyle ou alcoxy à chaîne droite ou ramifiée contenant chacun jusqu'à 8 atomes de carbone, ou bien
un groupe alkyle à chaîne droite ou ramifiée contenant jusqu'à 8 atomes de carbone et éventuellement substitué par un groupe phényle lui-même éventuellement substitué par des halogènes, des groupes cyano, nitro, trifluorométhyle, trifluorométhoxy, trifluorométhylthio, hydroxy ou par un groupe alkyle ou alcoxy à chaîne droite ou ramifiée contenant chacun jusqu'à 6 atomes de carbone,
et leurs sels.

2. Quinoléylméthoxyphénylacétamides à substituants hétérocycliques selon la revendication 1, pour lesquels :
A, B, D, E, G, L et M, ayant des significations identiques ou différentes, représentent chacun :
l'hydrogène, un groupe hydroxy, le fluor, le chlore, le brome, un groupe carboxy, nitro, trifluorométhyle, trifluorométhoxy, ou bien
un groupe alkyle ou alcoxy à chaîne droite ou ramifiée contenant chacun jusqu'à 6 atomes de carbone, ou bien
un groupe phényle lui-même éventuellement substitué par le fluor, le chlore, le brome, des groupes hy-

droxy, nitro ou cyano,
$R^1$ représente un groupe alkyle à chaîne droite ou ramifiée contenant jusqu'à 8 atomes de carbone et éventuellement substitué par des groupes phényle, cyclopentyle, cyclohexyle ou cycloheptyle,
ou bien un groupe cyclobutyle, cyclopentyle, cyclohexyle, cycloheptyle ou cyclooctyle,
$R^2$ représente l'hydrogène ou un groupe alkyle à chaîne droite ou ramifiée contenant jusqu'à 6 atomes de carbone,
T représente un groupe pyridyle, thiazolyle, tétrazolyle, pyrryle, furyle, thiényle, thiazolyle, thiadiazolyle, thiazolinyle ou imidazolyle éventuellement substitué par un groupe alkyle à chaîne droite ou ramifiée contenant jusqu'à 6 atomes de carbone, le fluor, le chlore, le brome, ou par un groupe de formules $NR^3R^4$ ou $-SO_2R^5$, dans lesquelles :
$R^3$ et $R^4$, ayant des significations identiques ou différentes, représentent chacun l'hydrogène ou un groupe alkyle à chaîne droite ou ramifiée contenant jusqu'à 6 atomes de carbone, et
$R^5$ représente un groupe cyclobutyle, cyclopentyle, cyclohexyle, cycloheptyle, cyclooctyle ou phényle éventuellement substitué par le fluor, le chlore, le brome, l'iode, un groupe cyano ou un groupe alkyle ou alcoxy à chaîne droite ou ramifiée contenant chacun jusqu'à 6 atomes de carbone, ou bien
un groupe alkyle à chaîne droite ou ramifiée contenant jusqu'à 6 atomes de carbone, éventuellement substitué par un groupe phényle lui-même éventuellement substitué par le fluor, le chlore, le brome, un groupe trifluorométhyle ou un groupe alkyle ou alcoxy à chaîne droite ou ramifiée contenant jusqu'à 4 atomes de carbone,
et leurs sels.

3. Quinoléylméthoxyphénylacétamides à substituants hétérocycliques selon la revendication 1, pour lesquels :
A, B, D, E, G, L et M, ayant des significations identiques ou différentes, représentent chacun :
l'hydrogène, un groupe hydroxy, le fluor, le chlore, le brome ou un groupe alkyle à chaîne droite ou ramifiée contenant jusqu'à 4 atomes de carbone,
$R^1$ représente un groupe alkyle à chaîne droite ou ramifiée contenant jusqu'à 6 atomes de carbone et éventuellement substitué par un groupe phényle ou cyclohexyle, un groupe cyclobutyle, cyclopentyle, cyclohexyle, cycloheptyle ou cyclooctyle,
$R^2$ représente l'hydrogène ou un groupe alkyle à chaîne droite ou ramifiée contenant jusqu'à 4 atomes de carbone,
T représente un groupe pyridyle, triazolyle, tétrazolyle, thiazolyle, thiadiazolyle ou thiazolinyle, éventuellement substitué par un groupe alkyle à chaîne droite ou ramifiée contenant jusqu'à 4 atomes de carbone, le fluor, le chlore, des groupes amino ou un groupe de formule $-SO_2R^5$, dans lequel :
$R^5$ représente un groupe cyclopentyle, cyclohexyle, cycloheptyle, cyclooctyle ou phényle éventuellement substitué par le fluor, le chlore, le brome, l'iode, des groupes méthoxy ou trifluorométhyle, ou bien
un groupe alkyle à chaîne droite ou ramifiée contenant jusqu'à 4 atomes de carbone et éventuellement substitué par un groupe phényle lui-même éventuellement substitué par le fluor, le chlore, le brome, des groupes méthyle ou méthoxy,
et leurs sels.

4. Quinoléylméthoxyphénylacétamides à substituants hétérocycliques selon la revendication 1, dans lesquels le groupe $-CHR^1-CO-NR^2-T$ est en position 4 par rapport au groupe quinoléylméthoxy.

5. Quinoléylméthoxyphénylacétamides à substituants hétérocycliques selon la revendication 1 pour le traitement de maladies.

6. Procédé de préparation des quinoléylméthoxyphénylacétamides à substituants hétérocycliques selon une des revendications 1 à 4, caractérisé en ce que l'on amide des composés de formule générale :

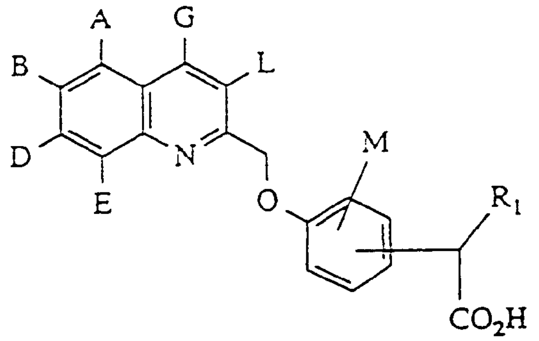

(II)

dans laquelle :
A, B, D, E, G, L, M et $R^1$ ont les significations indiquées ci-dessus, à l'aide de composés de formule générale III :

$H_2N$-T (III)

dans laquelle :
T a les significations indiquées ci-dessus,
dans des solvants inertes, en présence d'une base et/ou d'un produit auxiliaire et le cas échéant après activation de la fonction acide carboxylique,
et, dans le cas des énantiomères, on sépare au préalable les énantiomères purs des acides II,
la nature des substituants A, B, D, E, G, L, M et $R^1$ pouvant éventuellement être modifiée par des modes opératoires usuels.

7. Médicament contenant des quinoléylméthoxyphénylacétamides à substituants hétérocycliques selon la revendication 1.

8. Utilisation des quinoléylméthoxyphénylacétamides à substituants hétérocycliques selon la revendication 1 pour la préparation de médicaments.

9. Utilisation de quinoléylméthoxyphénylacétamides à substituants hétérocycliques selon la revendication 1 pour la préparation de médicaments inhibiteurs de la lipoxygénase.

**Revendications pour l'Etat contractant suivant : ES**

1. Procédé de préparation de quinoléylméthoxyphénylacétamides à substituants hétérocycliques de formule générale :

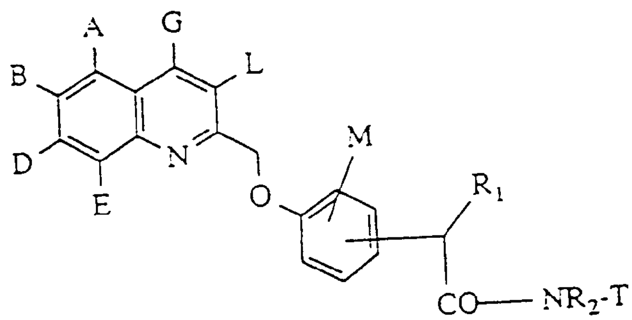

(I),

dans laquelle :
A, B, D, E, G, L et M, ayant des significations identiques ou différentes, représentent chacun :
l'hydrogène, un groupe hydroxy, un halogène, un groupe cyano, carboxy, nitro, trifluorométhyle, trifluorométhoxy, ou bien
un groupe alkyle ou alcoxy à chaîne droite ou ramifiée contenant chacun jusqu'à 8 atomes de carbone, ou bien
un groupe aryle en $C_6$-$C_{10}$ éventuellement substitué par des halogènes, des groupes hydroxy, nitro ou

cyano,
$R^1$ représente un groupe alkyle à chaîne droite ou ramifiée contenant jusqu'à 10 atomes de carbone et éventuellement substitué par un groupe aryle en $C_6$-$C_{10}$ ou par un groupe cycloalkyle en $C_3$-$C_8$, ou bien un groupe cycloalkyle ou -alcényle contenant 3 à 12 atomes de carbone,
$R^2$ représente l'hydrogène ou un groupe alkyle à chaîne droite ou ramifiée contenant jusqu'à 8 atomes de carbone,
T représente un hétérocycle saturé ou insaturé de 5 à 7 chaînons contenant jusqu'à 4 hétéroatomes choisis parmi S, O ou N, et condensé le cas échéant avec un hétérocycle ou un carbocycle saturé ou insaturé, ces cycles étant éventuellement substitués par des groupes alkyle à chaîne droite ou ramifiée contenant jusqu'à 8 atomes de carbone, des halogènes, des groupes benzyloxy ou un groupe de formules $-NR^3R^4$ ou $-SO_2R^5$, dans lesquelles :
$R^3$ et $R^4$, ayant des significations identiques ou différentes, représentent chacun l'hydrogène, un groupe phényle, benzyle ou un groupe alkyle à chaîne droite ou ramifiée contenant jusqu'à 8 atomes de carbone, et
$R^5$ représente un groupe cycloalkyle en $C_3$-$C_{12}$ ou aryle en $C_6$-$C_{10}$, portant éventuellement jusqu'à deux substituants identiques ou différents choisis parmi les halogènes, les groupes cyano, hydroxy, nitro, trifluorométhyle, trifluorométhoxy, trifluorométhylthio ou un substituant alkyle ou alcoxy à chaîne droite ou ramifiée contenant chacun jusqu'à 8 atomes de carbone, ou bien
un groupe alkyle à chaîne droite ou ramifiée contenant jusqu'à 8 atomes de carbone et éventuellement substitué par un groupe phényle lui-même éventuellement substitué par des halogènes, des groupes cyano, nitro, trifluorométhyle, trifluorométhoxy, trifluorométhylthio, hydroxy ou par un groupe alkyle ou alcoxy à chaîne droite ou ramifiée contenant chacun jusqu'à 6 atomes de carbone,
et leurs sels, caractérisé en ce que l'on amide des composés de formule générale :

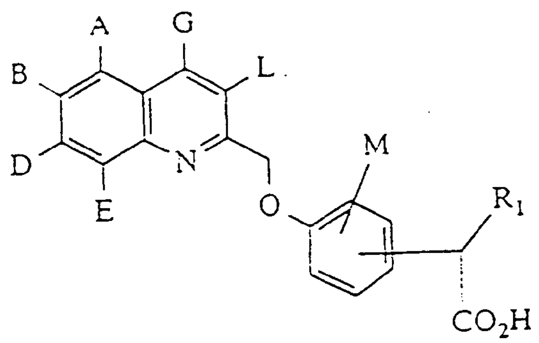

(II)

dans laquelle :
A, B, D, E, G, L, M et $R^1$ ont les significations indiquées ci-dessus, à l'aide de composés de formule générale III :

$$H_2N\text{-}T \qquad (III)$$

dans laquelle :
T a les significations indiquées ci-dessus,
dans des solvants inertes, en présence d'une base et/ou d'un produit auxiliaire et le cas échéant après activation de la fonction acide carboxylique,
et dans le cas des énantiomères, on sépare au préalable les énantiomères purs des acides II,
la nature des substituants A, B, D, E, G, L, M et $R^1$ pouvant éventuellement être modifiée par des modes opératoires usuels.